# FASCICULE DE BREVET EUROPEEN

(11) **EP 2 365 784 B1**
(45) Date de publication et mention de la délivrance du brevet: **31.05.2017**
(21) Numéro de dépôt: 09783765.2
(22) Date de dépôt: 06.10.2009
(51) Int. Cl.: A61C 1/08, A61C 1/10, A61C 1/12, A61B 17/16, B29C 33/48, B29C 33/50

(54) **PIECE A MAIN A USAGE DENTAIRE OU CHIRURGICAL ET SON PROCEDE DE FABRICATION**
HANDSTÜCK FÜR DENTALE ODER CHIRURGISCHE ANWENDUNGEN UND VERFAHREN ZU DESSEN HERSTELLUNG
HANDPIECE FOR DENTAL OR SURGICAL USE AND METHOD OF MAKING THE SAME

(30) Priorité: 07.10.2008 CH 15982008
(43) Date de publication de la demande: 21.09.2011
(73) Titulaire: Bien-Air Holding SA, 2500 Bienne 6 (CH)
(72) Inventeur: MAITRE, Luc, CH-2885 Epauvillers (CH)
(74) Mandataire: Giraud, Eric
(86) Numéro de dépôt international: PCT/EP2009/062937
(87) Numéro de publication internationale: WO 2010/040728

(56) Documents cités:
- EP-A1- 0 261 260
- EP-A2- 1 051 950
- EP-A2- 1 676 903
- WO-A2-2009/036462
- US-A- 5 387 098
- US-A1- 2006 210 948

## Description

La présente invention concerne une pièce à main à usage dentaire ou chirurgical. Plus précisément, la présente invention concerne un tel instrument à usage dentaire ou chirurgical dont le corps est réalisé dans un matériau léger et résistant.

Il existe essentiellement deux familles d'instruments à main à usage dentaire ou chirurgical. La première de ces familles regroupe ce que l'on appelle les turbines, à savoir des instruments dont l'outil, par exemple une fraise, est couplé à un moteur entraîné par de l'air sous pression. Un tel moteur peut tourner à plusieurs centaines de milliers de tours par minute avec une pression d'air typiquement de l'ordre de 2,5 à 3 bars. Une telle turbine comprend un manche qui permet à l'utilisateur de tenir son instrument en main. Ce manche est classiquement relié à une unité d'alimentation électrique et de fourniture de fluides (air, eau) via un raccord et un tuyau souple.

La seconde famille d'instruments à usage dentaire ou chirurgical regroupe ce que l'on appelle les pièces à main et les contre-angles. Les pièces à main et les contre-angles diffèrent pour l'essentiel en ce que le corps des pièces à main est sensiblement droit tandis que celui des contre-angles forme un angle en un endroit de sa longueur. Pour le reste, ces deux types d'instruments se ressemblent beaucoup. Un moteur électrique, logé dans le manche de la pièce à main ou du contre-angle et capable de tourner à plusieurs dizaines de milliers de tours par minute, est relié via un tuyau à raccord fixe ou tournant à l'unité d'alimentation.

Pour des raisons de commodité, on désignera dans ce qui suit les instruments à usage dentaire ou chirurgical tels que décrits ci-dessus par l'expression générique pièce à main, sachant que cette expression recouvre tout aussi bien les pièces à main proprement dites que les turbines et les contre-angles.

Les pièces à main se composent essentiellement d'un manche qui permet de les tenir en main et d'une tête qui porte notamment l'outil. Ces éléments sont classiquement fabriqués à partir d'un barreau de laiton ou d'acier inoxydable. Ils présentent des formes aussi bien extérieures qu'intérieures extrêmement complexes dont l'usinage (débitage, perçage, filetage, matriçage etc.) est long et coûteux et nécessite des machines-outils sophistiquées qui peuvent comprendre jusqu'à huit axes d'usinage différents. En outre, quelle que soit la sophistication des machines-outils utilisées, on est toujours limité par les formes que l'on peut donner à ces éléments.

On notera par ailleurs que les matériaux utilisés, laiton ou acier inoxydable, présentent des avantages mais aussi des inconvénients. Le laiton, par exemple, est un matériau très apprécié des constructeurs car il est facile à usiner. Par contre, le laiton présente l'inconvénient de s'oxyder facilement et doit donc subir des traitements de surface par dépôts de couches de matériaux inoxydables. Ces couches, relativement fragiles, ont tendance à s'user et à se rayer facilement, pouvant laisser apparaître par endroits la couche de laiton sous-jacente, ce qui donne aux utilisateurs le sentiment d'un instrument de qualité médiocre. En outre, certaines des couches contiennent du nickel au contact duquel les utilisateurs peuvent développer des allergies lorsque ces couches sont mises à nu.

Au contraire, l'acier inoxydable est, par définition, un matériau résistant bien à la corrosion et ne nécessitant donc pas de traitement spécifique de ce point de vue. Par ailleurs, l'acier inoxydable est plus léger que le laiton et permet donc de réaliser des pièces à main qui pèsent moins lourd dans la main de l'utilisateur, réduisant ainsi la fatigue que celui-ci peut ressentir après plusieurs heures de travail. Mais l'acier inoxydable présente l'inconvénient d'être difficile à usiner.

Pour remédier à ces problèmes, on a déjà réalisé certains composants de pièces à main (éléments pour le raccordement des pièces à main à l'unité d'alimentation et de contrôle, éléments isolants pour connecteurs électriques, ailettes de turbines etc.) par injection de matériaux plastiques. Certains des matériaux plastiques utilisés sont biocompatibles, ce qui permet de réaliser des composants destinés à entrer en contact avec l'utilisateur ou le patient. D'autre part, l'injection de matériaux plastiques permet d'obtenir des formes qu'il serait difficile, voire impossible à obtenir par usinage de pièces en laiton ou en acier inoxydable. Les formes qui peuvent être obtenues par injection d'un matériau plastique sont cependant limitées par les problèmes posés par le retrait du noyau ou insert après refroidissement de la matière plastique. Par exemple, il ne serait pas possible de retirer le noyau d'une pièce présentant une symétrie générale cylindrique et qui serait localement courbée. Par ailleurs, la gamme de matériaux plastiques qui s'offre au concepteur est très limitée en raison du manque de résistance de la plupart de ces matériaux aux cycles de stérilisation auxquels sont assujettis les instruments dentaires ou chirurgicaux. Enfin, en règle générale, les matériaux plastiques ne jouissent pas d'une bonne image auprès des utilisateurs.

Que les pièces à main soient réalisées en laiton ou en acier inoxydable ou bien encore par injection d'un matériau plastique, elles sont parfaitement rigides et transmettent donc toutes les vibrations engendrées par leur fonctionnement, ce qui est fatigant pour l'utilisateur et rend son geste imprécis.

Le document EP0261260 A1 décrit une perceuse chirurgicale avec un boîtier en composite epoxy/fibres de verre.

Il existait donc dans l'état de l'art un besoin pour un matériau permettant de combiner les avantages des matériaux plastiques (légèreté, liberté au niveau des formes, précision, répétitivité et rapidité de l'injection) et des matériaux métalliques (longévité, résistance mécanique), tout en ayant des propriétés d'absorption des vibrations engendrées par le fonctionnement des pièces à main.

La présente invention a pour but de répondre à cette attente en procurant une pièce à main à usage dentaire ou chirurgical, réalisé selon la revendication 1, comprenant des composants fixes et des composants mobiles, certains au moins de ces composants fixes sont réalisés en un matériau composite résultant de l'association d'au moins deux matériaux de base distincts, à savoir une matrice en résine époxy et un renfort.

La matrice est obtenue à l'aide d'une résine époxy à deux composants et le renfort est choisi dans le groupe comprenant les fibres ou particules de carbone, de verre, de bore, de kevlar, de céramique, d'aramide, de nylon, d'acier ou de tungstène.

Dans une pièce en matériau composite, la matrice en résine époxy et le renfort sont mis en contact intime à une échelle macroscopique, la matrice conférant à la pièce la forme voulue et transmettant au renfort (fibres ou particules) les sollicitations mécaniques auxquelles est soumise ladite pièce, ce qui permet d'améliorer certaines caractéristiques du matériau de renfort.

Une cause importante de fatigue chez les utilisateurs de pièces à main chirurgicales ou dentaires est liée aux vibrations engendrées par le fonctionnement de telles pièces à main. Or, on s'est rendu compte que des manches de pièces à main réalisés en résine époxy, outre le fait qu'ils sont très résistants, présentent une certaine souplesse et sont tout à fait à-même d'absorber les vibrations engendrées par le fonctionnement des pièces à main. Une pièce à main équipée d'un tel manche par exemple en fibre de carbone va donc moins vibrer, ce qui permet à son utilisateur de moins se fatiguer et d'avoir un geste plus précis.

Les composants fixes d'une pièce à main chirurgicale ou dentaire comprennent notamment la tête et le manche de telles pièces à main ainsi que les pièces de raccord fixe ou tournant pour la connexion de ces pièces à main sur une unité d'alimentation et de commande mieux connue sous sa dénomination anglo-saxonne « unit ». Les composants mobiles comprennent entre autres les moteurs de turbines, les entraîneurs et plus généralement les chaînes de transmission cinématiques pour transmettre le couple moteur aux outils et certains composants des pièces à main ou contre-angles.

Les matières plastiques semblaient ouvrir des perspectives prometteuses dans le domaine de la fabrication de composants pour pièces à main dentaires ou chirurgicales, mais on a été très vite confronté aux limites de l'injection de telles matières. En effet, pour pouvoir injecter une pièce en matière plastique, on doit disposer d'une part d'un moule qui reprend les formes extérieures de la pièce à injecter, et d'autre part d'un noyau ou insert rigide qui définit les formes intérieures de cette pièce. Après injection de la matière plastique dans le volume laissé libre entre le moule et le noyau, on refroidit la pièce puis on la démoule et on retire le noyau. C'est cette dernière étape de retrait du noyau qui pose problème. Effectivement, on ne peut pas envisager de réaliser des pièces présentant par exemple localement une réduction de diamètre ou une portion coudée car, dans un tel cas, il ne serait pas possible de retirer le noyau rigide après refroidissement de la pièce. Or, il existe un besoin dans la technique pour des pièces à main présentant des formes de fantaisie ou des formes ergonomiques qui n'a pas été satisfait jusqu'à ce jour dans la mesure où les forme accessibles par usinage de pièces en laiton ou en acier inoxydable ou bien par injection plastique sont des formes essentiellement cylindriques.

La présente invention, en enseignant de réaliser des éléments de pièces à mains chirurgicales ou dentaires tels que des têtes ou des manches en résine époxy permet de surmonter toutes ces limitations. En effet, l'élément déterminant dans la technique de fabrication de composants en résine époxy réside dans l'utilisation d'un noyau ou insert réalisé en un matériau compressible tel que du silicone. Plus précisément, dans le cas où l'on souhaite par exemple réaliser une pièce en fibres de carbone, le noyau compressible qui définit les formes intérieures de la pièce à réaliser est enveloppé dans une natte ou tressage en fibres de carbone puis introduit dans un moule qui reprend quand à lui les formes extérieures de la pièce à fabriquer. De la résine époxy est ensuite introduite dans l'interstice laissé vacant entre le moule et le noyau et vient imprégner la fibre de carbone. Lorsque la résine a durci, on démoule la pièce et on retire le noyau. Comme celui-ci est compressible, il est apte à se déformer et peut donc franchir sans difficulté un point où la pièce présente par exemple une zone de rétrécissement ou une portion coudée. La réalisation en un matériau composite d'éléments pour des pièces à main à usage chirurgical ou dentaire permet ainsi de s'affranchir de presque toutes les contraintes en termes de forme des éléments à réaliser et de proposer de telles pièces à main avec, par exemple, des manches ayant des formes de fantaisie ou présentant une ergonomie facilitant leur tenue en main. On notera cependant que la fabrication d'un manche de pièce à main en fibre de carbone au moyen de la technique décrite ci-dessus nécessite beaucoup plus de soins et d'attention que la fabrication d'un tel manche par exemple par injection d'un matériau plastique. L'étape au cours de laquelle le noyau est enveloppé dans la natte ou tressage en fibres de carbone requiert en particulier une grande vigilance. Il faut en effet veiller à ce que la natte en fibres de carbone épouse au plus près les formes du noyau pour être assuré de respecter non seulement la géométrie intérieure de la pièce résultante, mais aussi l'épaisseur de l'interstice qui sépare le noyau enveloppé dans sa natte du moule et qui détermine l'épaisseur de la pièce résultante et donc le respect des cotes dimensionnelles.

Réaliser des pièces à main en matériau composite permet également d'effectuer des gains en poids. Ainsi, par exemple, un manche réalisé en laiton qui pèse 14g ne pèsera plus que 4g lorsqu'il est réalisé en fibre de carbone. On réalise ainsi un gain de poids de 10g, ce qui représente un gain de l'ordre de 15% sur le poids total d'un instrument qui est typiquement de l'ordre de 70g. On comprendra aisément qu'une pièce à main plus légère est moins fatigante pour son utilisateur qui doit parfois la tenir en main plusieurs heures par jour.

L'invention concerne également un noyau selon la revendication 5. Ce noyau comporte des moyens de réception dans lesquels vont être logés, avant moulage, un ou plusieurs conduits pour l'amenée de l'air et/ou de l'eau et/ou un guide de lumière pour l'amenée de la lumière au niveau de la tête de la pièce à main. Préférentiellement, les moyens de réception sont des gorges dans lesquelles vont être logés par exemple des conduits pour l'amenée de l'air et de l'eau, et un barreau de verre ou bien un faisceau de fibres optiques pour l'amenée de la lumière au niveau de la tête de la turbine. Grâce à cette technique, on peut réaliser des pièces aux formes très complexes sans avoir besoin de se soucier de la façon dont ou pourrait manuellement introduire les divers conduits et guides optiques dans une pièce à main équivalente réalisée en laiton ou en acier inoxydable. Après avoir inséré les divers conduits et guides de lumière dans les gorges, on emmaillote le noyau dans un tressage par exemple de fibres de carbone, puis on introduit l'ensemble dans le moule. On imprègne ensuite le tressage de fibres au moyen d'une résine époxy que l'on laisse durcir, puis on démoule la pièce. Les conduits et guides optiques sont alors emprisonnés dans la résine.

D'autres caractéristiques et avantages de l'invention ressortiront plus clairement de la description détaillée qui suit d'un mode de réalisation de la pièce à main selon l'invention, cet exemple étant donné à titre purement illustratif et non limitatif seulement en liaison avec le dessin annexé sur lequel :
- la figure 1 est une vue en coupe d'une pièce à main du type turbine conforme à la présente invention ;
- la figure 2 est une vue en coupe longitudinale d'un moule illustrant le procédé de fabrication d'un manche en fibre de carbone selon l'invention, et

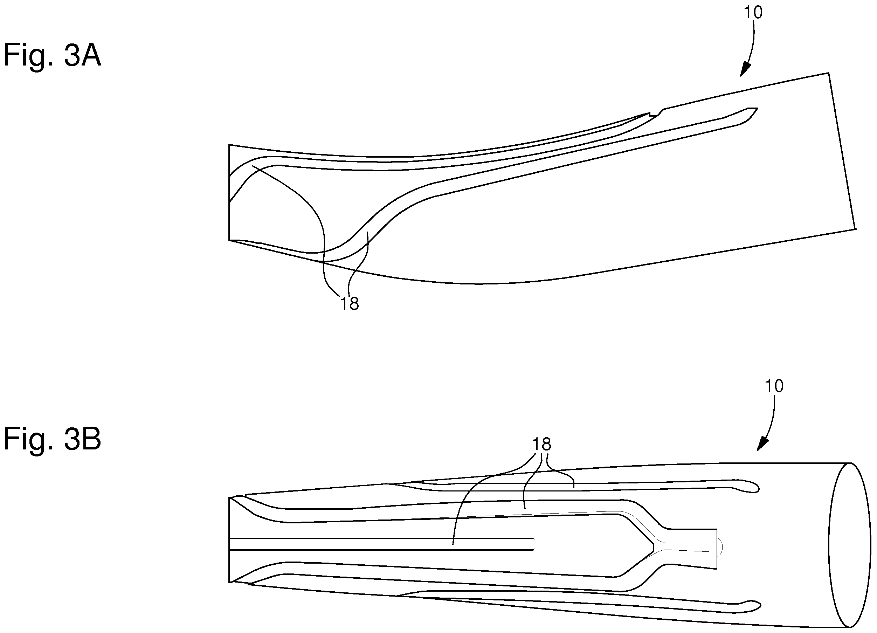

- la figure 3 est une vue en perspective d'une variante de réalisation d'un noyau pour la fabrication d'un manche en fibre de carbone.

La présente invention procède de l'idée générale inventive consistant à réaliser certains composants au moins d'une pièce à main à usage dentaire ou chirurgical en un matériau composite résultant de l'association d'au moins deux matériaux de base distincts, à savoir une matrice en résine époxy et un renfort. La plupart des pièces à main disponibles sur le marché sont réalisées en laiton ou en acier inoxydable, très rarement par injection d'un matériau plastique. Or, de telles pièces à main sont parfaitement rigides et transmettent donc toutes les vibrations engendrées par leur fonctionnement, ce qui est fatigant pour l'utilisateur et rend son geste imprécis. Or, on s'est rendu compte que des manches de pièces à main réalisés en résine époxy présentaient une certaine souplesse et étaient tout à fait à-même d'absorber les vibrations, ce qui est plus reposant pour l'utilisateur et lui permet d'améliorer la précision de son geste. En outre, une pièce à main dont par exemple le manche est réalisé en résine époxy est plus résistante et plus légère. Enfin, en réalisant le manche d'une pièce à main en résine époxy, on peut prévoir des formes de fantaisie ou bien des formes ergonomiques facilitant la tenue en main du manche. En effet, le noyau ou insert de moulage est réalisé en un matériau élastomérique compressible, ce qui permet de le retirer après moulage même si le manche présente localement des rétrécissements de diamètre ou des formes courbes car le noyau pourra se comprimer en passant à hauteur des zones de diamètre réduit.

Par souci de clarté, la présente invention va être décrite en liaison avec la réalisation d'un manche de pièce à main à usage dentaire ou chirurgical en fibre de carbone imprégnée de résine époxy. Il doit néanmoins être bien entendu que cet exemple est donné à titre purement illustratif et non limitatif seulement et que, selon une variante de l'invention, un tel manche pourra être réalisé de manière similaire au moyen de fibres de verre, de bore, de kevlar, de céramique, d'aramide, de nylon, d'acier ou de tungstène imprégnées de résine époxy. De même, selon une autre variante de l'invention, les composants fixes d'une pièce à main objet de l'invention peuvent être réalisés au moyen d'une ou de plusieurs couches de fibres imprégnées.

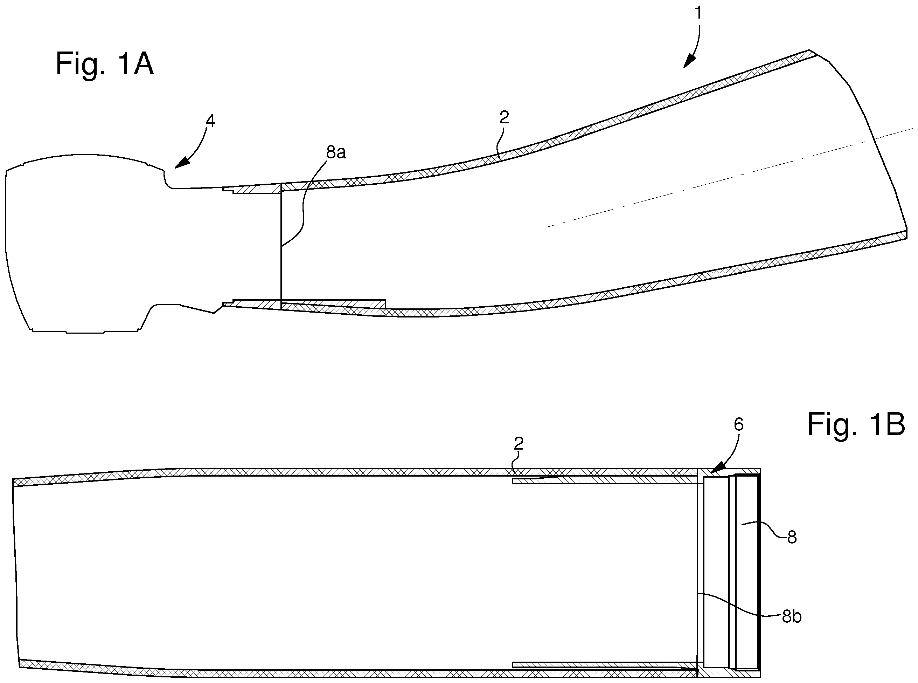

La figure 1 est une vue en coupe longitudinale d'une turbine. Désignée dans son ensemble par la référence numérique générale 1, cette turbine comprend un manche 2 réalisé en fibre de carbone sur lequel se raccordent une tête 4 et un embout 6 pour le montage d'une prise femelle 8 sur laquelle se raccordera un raccord rapide (non représenté) pour l'amenée des fluides de travail (air et eau) ainsi que de l'électricité. La tête 4 et l'embout 6 pourront par exemple être réalisés en inox pour trancher avec la couleur noire de la fibre de carbone et procurer ainsi un effet esthétique intéressant au niveau des extrémités 8a et 8b du manche 2 sur lesquelles ils se raccordent.

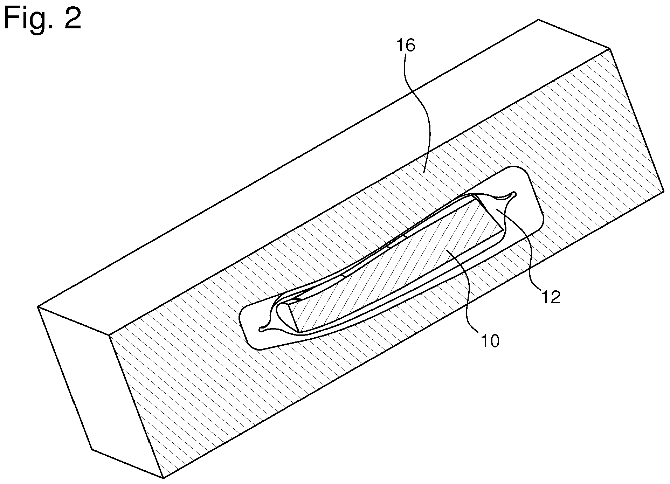

Un exemple de mise en oeuvre du procédé de fabrication d'un manche en fibre de carbone selon l'invention est donné en liaison avec la figure 2 annexée à la présente demande de brevet. Un noyau ou insert 10 réalisé dans un matériau compressible tel que du silicone et dont les formes extérieures correspondent au profil intérieur recherché du manche à réaliser est emmailloté dans une natte 12 en fibres de carbone. Le noyau 10 ainsi enveloppé dans la natte 12 de fibres de carbone est disposé à l'intérieur d'un moule 16 dont la surface intérieure correspond aux formes extérieures recherchées du manche 2 à réaliser. L'épaisseur de la natte 12 de fibres de carbone est typiquement de trois dixièmes de millimètre et le jeu entre le noyau 10 et le moule 16 est de l'ordre d'un dixième de millimètre. On fait ainsi avancer dans l'espace laissé libre entre le noyau 10 et le moule 16 une résine époxy qui va venir imprégner la natte 12 de fibres de carbone. On comprendra que, de manière équivalente, on peut utiliser une natte de fibres par exemple de verre ou de bore en remplacement de la natte de fibres de carbone.

Lorsque la résine époxy a durci, on démoule la pièce ainsi obtenue. Comme le noyau 10 est compressible, il est susceptible de se déformer pour passer les endroits où le manche 2 est courbe ou présente des réductions de diamètre. Le manche 2 ainsi obtenu va présenter un état de surface induit par l'état de la surface intérieure du moule 16. Si la surface intérieure du moule 16 présente un état poli-miroir, la surface du manche 2 sera lisse et brillante. Au contraire, si la surface intérieure du moule 16 présente par endroits des zones rugueuses, la surface du manche 2 présentera aux endroits correspondants un état de surface mat et rugueux. Ces surfaces peuvent par exemple définir pour l'utilisateur des zones de préhension du manche 2.

Selon une variante de l'invention, on prévoit sur le noyau 10 des moyens de réception, par exemple sous la forme de gorges 18, dans lesquelles vont être logés par exemple des conduits pour l'amenée de l'air et de l'eau, un barreau de verre ou bien un faisceau de fibres optiques pour l'amenée de la lumière au niveau de la tête 4 de la turbine 1. Lorsque les divers conduits et guides optiques sont insérés dans les gorges 18, on enfile le noyau 10 dans la natte 12 de fibres de carbone. Les conduits et guides de lumière ne gênent par l'engagement de la natte 12 de fibres de carbone dans la mesure où celle-ci peut être étirée jusqu'à deux fois sa taille normale. La suite des opérations est identique à celle décrite ci-dessus. On introduit le noyau 10 avec sa natte 12 de fibres de carbones dans un moule que l'on referme. On imprègne les fibres avec une résine époxy que l'on laisse durcir, puis on démoule la pièce et on la découpe aux formes et aux dimensions voulues. Finalement, on retire le noyau. Les conduits d'air et/ou d'eau ainsi que le guide optique se retrouvent emprisonnés dans la résine époxy. On peut ainsi, par cette technique, fabriquer une pièce à main aux formes très complexes sans avoir à se soucier de la façon dont on pourrait engager manuellement les divers conduits et guides optiques après usinage d'une pièce à main équivalente en laiton ou en acier inoxydable.

## Revendications

1. Procédé de fabrication d'une pièce à main à usage dentaire ou chirurgical comprenant des composants fixes (2) et des composants mobiles, les composants fixes comprenant la tête et le manche (2) de telles pièces à main ainsi que les pièces de raccord fixe ou tournant pour la connexion de ces pièces à main (1) sur une unité d'alimentation et de commande, certains au moins de ces composants fixes (2) étant réalisés en un matériau composite résultant de l'association d'au moins deux matériaux de base distincts, à savoir une matrice en résine époxy et un renfort sous la forme d'une natte ou tressage (12) en fibres, le matériau composite étant déposé selon une ou plusieurs couches, le procédé étant **caractérisé en ce que** un noyau ou insert (10) réalisé en un matériau compressible qui définit les formes intérieures du composant fixe de la pièce à main (1) à réaliser est enveloppé dans la natte ou tressage (12) en fibres, puis introduit dans un moule (16) qui reprend quant à lui les formes extérieures du composant fixe de la pièce à main (1) à fabriquer, la résine époxy étant ensuite introduite dans un interstice laissé vacant entre le moule (16) et le noyau (10) et venant imprégner la natte ou tressage (12) en fibres, le composant fixe de la pièce à main (1) étant finalement démoulé lorsque la résine a durci et le noyau (10) étant retiré.

2. Pièce à main à usage dentaire ou chirurgical comprenant des composants fixes et des composants mobiles, réalisée par le procédé selon la revendication 1.

3. Pièce à main selon la revendication 2, **caractérisée en ce que** la matrice est une résine époxy à deux composants, et **en ce que** le renfort est choisi dans le groupe comprenant les fibres de carbone, de verre, de bore, de kevlar, de céramique, d'aramide, de nylon, d'acier ou de tungstène.

4. Pièce à main selon la revendication 2, **caractérisée en ce que** le manche (2) présente des surfaces lisses et des surfaces mates.

5. Noyau ou insert (10) pour la mise en oeuvre d'un procédé de fabrication d'une pièce à main (1) à usage dentaire ou chirurgical selon la revendication 1, **caractérisé en ce que** le noyau ou insert (10) est réalisé en un matériau compressible et **en ce qu'**il comporte des moyens de réception dans lesquels vont être logés, avant moulage, un ou plusieurs conduits pour l'amenée de l'air et/ou de l'eau et/ou un guide de lumière pour l'amenée de la lumière au niveau de la tête (4) de la pièce à main (1).

6. Noyau ou insert (10) selon la revendication 5, **caractérisée en ce que** les moyens de réception sont des gorges (18).

## Patentansprüche

1. Verfahren zum Herstellen eines Handstücks zur zahnmedizinischen oder chirurgischen Anwendung, umfassend feste Komponenten (2) und bewegliche Komponenten, wobei die festen Komponenten den Kopf und den Griff (2) solcher Handstücke sowie die Teile für eine feste oder drehbare Verbindung, um diese Handstücke (1) mit einer Versorgungs- und Steuereinheit zu verbinden, umfassen, wobei mindestens bestimmte feste Komponenten (2) aus einem Verbundwerkstoff gebildet sind, der sich aus der Zusammenfügung von mindestens zwei verschiedenen Ausgangsmaterialien, nämlich einem Epoxidharz-Grundstoff und einer Verstärkung in Form einer Fasermatte oder eines Fasergeflechts (12) ergeben, wobei der Verbundwerkstoff in einer oder in mehreren Schichten abgelagert ist und das Verfahren **dadurch gekennzeichnet ist, dass** ein aus einem komprimierbaren Material gebildeter Kern oder Einsatz (10), der die inneren Formen der festen Komponente des zu bildenden Handstücks (1) definiert, von einer Fasermatte oder einem Fasergeflecht (12) umwickelt wird, dann in eine Gießform (16) eingeführt wird, die ihrerseits die äußeren Formen der festen Komponente des herzustellenden Handstücks (1) nachbildet, anschließend das Epoxidharz in den zwischen der Gießform (16) und dem Kern (10) zurückbleibenden Zwischenraum eingeleitet wird und die Fasermatte oder das Fasergeflecht (12) imprägniert, und schließlich die feste Komponente des Handstücks (1) aus der Form entnommen wird, wenn das Harz gehärtet ist, und der Kern (10) herausgezogen wird.

2. Handstück zur zahnmedizinischen oder chirurgischen Anwendung, umfassend feste Komponenten und bewegliche Komponenten, das durch das Verfahren nach Anspruch 1 gebildet ist.

3. Handstück nach Anspruch 2, **dadurch gekennzeichnet, dass** der Grundstoff ein Zweikomponenten-Epoxidharz ist und dass die Verstärkung aus der Gruppe gewählt ist, die Fasern aus Kohlenstoff, Glas, Bor, Kevlar, Keramik, Aramid, Nylon, Stahl oder Wolfram enthält.

4. Handstück nach Anspruch 2, **dadurch gekennzeichnet, dass** der Griff (2) glatte Oberflächen und raue Oberflächen aufweist.

5. Kern oder Einsatz (10) für die Ausführung eines Verfahrens zur Herstellung eines Handstücks (1) zur zahnmedizinischen oder chirurgischen Anwendung nach Anspruch 1, **dadurch gekennzeichnet, dass** der Kern oder Einsatz (10) aus einem komprimierbaren Material gebildet ist und **dadurch gekennzeichnet, dass** er Aufnahmemittel umfasst, in denen vor dem Gießen eine oder mehrere Leitungen für die Zuführung von Luft und/oder Wasser und/oder ein Lichtleiter, um auf Höhe des Kopfes (4) des Handstücks (1) Licht einzuleiten, aufgenommen werden.

6. Kern oder Einsatz (10) nach Anspruch 5, **dadurch gekennzeichnet, dass** die Aufnahmemittel Kehlen (18) sind.

## Claims

1. Method of manufacturing a surgical or dental handpiece including fixed components (2) and moveable components, the fixed components including the head and the handle (2) of said handpiece and the fixed or rotating connector parts for connecting said handpieces (1) to a supply and control unit, at least some of said fixed components (2) being made from a composite material resulting from the association of at least two distinct basic materials, namely an epoxy resin matrix and a reinforcing agent in the form of a fibre mat (12), the composite material being deposited in one or several layers, the method being **characterized in that** a core or insert (10), made from a compressible material which defines the internal shapes of the fixed component of the handpiece (1) to be manufactured is wrapped in the fibre mat (12), then arranged inside a mould (16) which takes the external shapes of the fixed component of the handpiece (1) to be manufactured, the epoxy resin being then moved into a gap left free between mould (16) and core (10) and impregnating the fibre mat (12), the fixed component of the handpiece (1) being finally taken out of the mould when the resin has hardened and the core (10) being removed.

2. Dental or surgical handpiece including fixed components and moveable components, resulting from the method according to claim 1.

3. Handpiece according to claim 2, **characterized in that** the matrix is an epoxy resin with two components, and **in that** the reinforcing agent is selected from the group containing fibres of carbon, glass, boron, kevlar, ceramic, aramid, nylon, steel or tungsten.

4. Handpiece according to claim 3, **characterized in that** the handle (2) has smooth surfaces and matt surfaces.

5. Core or insert (10) for implementing a method of manufacturing a dental or surgical handpiece (1) according to claim 1, **characterized in that** the core or insert (1) is made of a compressible material and **in that** it includes receiving means in which one or several conduits will be housed, prior to moulding, for the air and/or water supply and/or a light guide supplying light to the head (4) of the handpiece (1).

6. Core or insert (10) according to claim 5, **characterized in that** the receiving means is grooves (18).
